(19) Europäisches Patentamt
European Patent Office
Office européen des brevets

(11) **EP 4 194 085 A1**

(12) **EUROPÄISCHE PATENTANMELDUNG**

(43) Veröffentlichungstag:
**14.06.2023 Patentblatt 2023/24**

(21) Anmeldenummer: **22211067.8**

(22) Anmeldetag: **02.12.2022**

(51) Internationale Patentklassifikation (IPC):
**B01J 21/12** (2006.01) **B01J 23/755** (2006.01)
**B01J 35/00** (2006.01) **B01J 37/02** (2006.01)
**B01J 37/03** (2006.01) **B01J 37/08** (2006.01)
**B01J 37/10** (2006.01)

(52) Gemeinsame Patentklassifikation (CPC):
**B01J 37/08; B01J 21/12; B01J 23/755;**
**B01J 35/002; B01J 35/0026; B01J 37/0201;**
**B01J 37/0211; B01J 37/033; B01J 37/088;**
**B01J 37/10;** B01J 21/08

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**KH MA MD TN**

(30) Priorität: **08.12.2021 EP 21213152**

(71) Anmelder: **Evonik Operations GmbH**
**45128 Essen (DE)**

(72) Erfinder:
• **Knossalla, Johannes**
**46514 Gahlen (DE)**
• **Quandt, Thomas**
**45772 Marl (DE)**
• **Franke, Robert**
**45772 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **VERFAHREN ZUR HERSTELLUNG EINES OLIGOMERISIERUNGSKATALYSATORS MIT EINEM HYDROTHERMALEN BEHANDLUNGSSCHRITT**

(57) Die vorliegende Erfindung betrifft ein Verfahren zur Herstellung eines Oligomerisierungskatalysators, welches einen Schritt zur hydrothermalen Behandlung aufweist. Ein weiterer Gegenstand der vorliegenden Erfindung ist ein durch das Verfahren hergestellter Oligomerisierungskatalysator und dessen Einsatz in der Oligomerisierung von C2- bis C12-Olefinen.

**EP 4 194 085 A1**

**Beschreibung**

Gegenstand der Erfindung ist ein Verfahren zur Herstellung eines Oligomerisierungskatalysators, welches einen Schritt zur hydrothermalen Behandlung aufweist. Ein weiterer Gegenstand der vorliegenden Erfindung ist ein durch das Verfahren hergestellter Oligomerisierungskatalysator und dessen Einsatz in der Oligomerisierung von C2- bis C12-Olefinen.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt. Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Bei den heterogen katalysierten Verfahren ist die Oligomerisierung an sauren Oligomerisierungskatalysatoren lange bekannt. Technisch werden z. B. Zeolithe oder Phosphorsäure auf einem Support eingesetzt. Hierbei werden Isomerengemische von verzweigten Olefinen erhalten. Für die nicht-saure, heterogen katalysierte Oligomerisierung von Olefinen, mit hoher Dimerenselektivität werden in der Technik häufig Nickelverbindungen auf einem Support, beispielsweise Aluminiumsilikat, eingesetzt, beispielsweise in der EP 3 542 898 A1.

Für die Herstellung von Oligomerisierungskatalysatoren sind verschiedene Verfahren im Stand der Technik bekannt. Ein wesentlicher Bestandteil bei der Herstellung ist eine innige Vermischung einer Nickelquelle und einem Aluminiumsilikat. Die weitere Formgebung einer solchen Mischung kann über die unterschiedlichsten in der Technik bekannten Verfahren erfolgen. Die Mischung kann zunächst zu einem Pulver verarbeitet werden, welches dann über dem Fachmann bekannte Verfahren verformt, z.B. Extrusion, Pelletierung, Kompaktierung, Tablettierung, Sprühgranulation, oder auf einem bestehenden Formkörper z.B. mittels Coating aufgebracht werden kann. Alternativ kann die Mischung durch geeignete Verfahren z.B. Granulation oder Knetung, direkt verdichtet werden und über bekannte Verfahren, wie Granulation, Extrusion oder Pelletierung, in Formkörper überführt werden.

Die EP 3 542 898 A1 offenbart beispielsweise ein Verfahren zur Herstellung eines Oligomerisierungskatalysators, bei dem die entsprechenden Einzelkomponenten gemischt und granuliert werden und die granulierte Mischung anschließend mittels Imprägnierung mit Nickel beaufschlagt wird. Durch anschließende Trocknung und Kalzination wird der finale Katalysator hergestellt. Bei den aus dem Stand der Technik bekannten Verfahren zur Herstellung eines Katalysators kann es dabei jedoch zu Problemen mit der Festigkeit des Katalysators bei seinem bestimmungsgemäßen Einsatz kommen. In Bezug zu Oligomerisierungskatalysatoren gibt es darüber hinaus einen steten Wunsch nach Verbesserung der Katalysatoreigenschaften, beispielsweise der Katalysatorperformance.

Der vorliegenden Erfindung lag die Aufgabe zugrunde ein Verfahren zur Herstellung eines Oligomerisierungskatalysators mit verbesserten Eigenschaften bereitzustellen. Dieser Oligomerisierungskatalysator im Vergleich mit bekannten Systemen sollte insbesondere eine erhöhte Festigkeit und eine verbesserte Katalysatorperformance aufweisen.

Es hat sich überraschend gezeigt, dass die zugrundeliegende Aufgabe mit einem Verfahren zur Herstellung eines Oligomerisierungskatalysators gelöst werden konnte, bei dem vor dem Trocknen eine zusätzliche hydrothermale Behandlung erfolgt. Die hydrothermale Behandlung wird erfindungsgemäß als ein Schritt verstanden, bei dem ein feuchtes Material (Mischung mit einem gewissen Flüssiganteil) vor der eigentlichen Trocknung und der abschließenden Kalzination einer erhöhten Temperatur (T > Raumtemperatur) ausgesetzt wird, wobei der Wassergehalt und der optionale Ammoniakgehalt für eine gewisse Zeit auf einem hohen Niveau bleibt und weniger stark reduziert wird als bei der Trocknung. Das erfindungsgemäße Verfahren ist daher ein Verfahren zur Herstellung eines Nickel-enthaltenden Oligomerisierungskatalysators, welches die folgenden Schritte umfasst:

a) Herstellung einer Mischung umfassend amorphes oder zeolithisches Aluminiumsilikat, welches 10 bis 65 Gew.-% Aluminiumoxid und 35 bis 90 Gew.-% Siliciumdioxid umfasst, optional einen Al-haltigen und Si-freien Binder oder einen Al-freien und Si-haltigen Binders, eine Nickelquelle, die eine Nickelverbindung umfasst, und Wasser, wobei der Flüssiganteil in der Mischung das 1,5-fache des TPV (total pore volume) des Aluminiumsilikats und des optionalen Binders, vorzugsweise das 1,3-fache des TPV, besonders bevorzugt das 1,25-fache des TPV nicht überschreitet, wobei das TPV mittels Stickstoffadsorption gemäß DIN 66134 bestimmt wird;

b) hydrothermale Behandlung der so hergestellten Mischung, wobei die hydrothermale Behandlung bei einer Temperatur im Bereich von 75 °C bis 180 °C, vorzugsweise 80 °C bis 150 °C erfolgt;

c) Trocknen des aus Schritt b) erhaltenen Mischung, wobei die Trocknungsrate in Schritt c) höher ist als in Schritt b);

d) Kalzinieren der in Schritt c) getrockneten Mischung zur Herstellung des Oligomerisierungskatalysators.

Im ersten Schritt a) wird die ursprüngliche Mischung hergestellt, die neben dem Trägermaterial Aluminiumsilikat, eine Nickelquelle und Wasser enthält. Die Mischung kann zusätzlich Ammoniak enthalten. Die Mischung kann weiterhin einen Binder oder andere Stoffe, beispielsweise andere Metalloxide, enthalten. In einer bevorzugten Ausführungsform der vorliegenden Erfindung umfasst die Mischung, die in Schritt a) hergestellt wird, einen Al-haltigen und Si-freien Binder oder einen Al-freien und Si-haltigen Binder.

Bei der Herstellung der Mischung in Schritt a) ist es wichtig, dass auf den Flüssiganteil in der Mischung geachtet wird. Dieser Flüssiganteil in der Mischung darf das 1,5-fache des TPV (total pore volume), vorzugsweise das 1,3-fache des TPV, besonders bevorzugt das 1,25-fache des TPV nicht überschreiten. TPV meint das gesamte Porenvolumen von Aluminiumsilikat und des optionalen Binders in der Mischung. Das Porenvolumen TPV kann beispielsweise mittels Stickstoffadsorption gemäß DIN 66134 (Stand: Februar 1998). Es soll also maximal etwas mehr Flüssigkeit (Wasser und optional Ammoniak) zugegeben werden als für die Absättigung der Poren benötigt wird. Ziel der Herstellung in Schritt a) ist somit eine feuchte Mischung, die noch Wasser aus der Herstellung enthält. Die feuchte Mischung wurde dementsprechend keine Trocknung unterworfen. Beispiele für unter den Begriff feuchte Mischung fallende Mischungen sind (feuchte) Pulver, Granulate oder plastisch verformbare Massen. In Übereinstimmung mit der etablierten Definition wird als plastisch verformbar Masse vorliegend verstanden, dass sich das entsprechende Material nach Überwinden einer Fließgrenze bleibend verformt, ohne dass der Zusammenhalt der die Masse bildenden Teilchen verloren geht. Bevorzugt wird in Schritt a) ein Granulat hergestellt, also eine Granulation mit der Mischung durchgeführt.

Es wird daher in Schritt a) explizit keine Suspension, kein Slurry und keine Lösung hergestellt, die sich alle jeweils über einen deutlich höheren Flüssiganteil definieren lassen. Daher ist auch kein Trennschritt notwendig, um Flüssigkeit abzutrennen und es fällt auch kein entsprechender flüssiger Abfall an. Die noch vorhandene Flüssigkeit (Wasser und optional Ammoniak) wird spätestens bei der Trocknung in Schritt c) in ausreichendem Umfang entfernt.

Das bei der Herstellung der Mischung in Schritt a) verwendete Aluminiumsilikat meint silikatische Materialien aus Aluminium, Silicium und Sauerstoff, die aus $SiO_4$- und $AlO_4$-Tetraedern aufgebaut sind. Andere gebräuchliche Begriffe für Aluminiumsilikate sind beispielsweise Alumosilicat, Silica-Alumina oder Aluminosilicat. Das in der Mischung in Schritt a) verwendete Aluminiumsilikat kann ein amorphes Alumosilicat oder ein zeolithisches Alumosilicat sein. Mit "amorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Aluminiumsilikat kleine kristalline Domänen aufweist. Das amorphe Aluminiumsilikat ist dennoch kein kristallines Material, also auch kein zeolithisches Material.

Das Aluminiumsilikat umfasst erfindungsgemäß 10 bis 65 Gew.-% Aluminiumoxid und 35 bis 90 Gew.-% Siliciumdioxid, vorzugsweise 10 bis 45 Gew.-% Aluminiumoxid und 55 bis 90 Gew.-% Siliciumdioxid. Die Angabe "Gew.-%" bezieht sich im Rahmen der vorliegenden Erfindung auf die Zusammensetzung ohne den Glühverlust, der bei kommerziell erhältlichen Produkten angegeben wird.

Der in Schritt a) ebenfalls eingesetzte Binder kann in einer Ausführungsform ein Al-haltiger und Si-freier Binder (Si-frei bedeutet: <0,1 Gew.-% Si in der Gesamtzusammensetzung des Binders) sein. Der Al-haltige und Si-freie Binder ist vorzugsweise ein oxidisches Aluminiummaterial, vorzugsweise Aluminiumoxid, Aluminiumhydroxid, oder Aluminiumoxid-hydroxid, besonders bevorzugt Boehmit. Der Al-haltige und Si-freie Binder liegt weiterhin bevorzugt nicht in fester Form, sondern in gelöster Form, besonders bevorzugt als kolloidale Lösung, vor. Das Lösemittel, indem der Al-haltige und Si-freie Binder, vorzugsweise Aluminiumoxid, Aluminiumhydroxid, oder Aluminiumoxid-hydroxid, besonders bevorzugt Boehmit, in gelöster Form, vorzugsweise als kolloidale Lösung vorliegt, ist in einer bevorzugten Ausführungsform eine 1 Gew.-%ige Salpetersäurelösung. Der Al-haltige und Si-freie Binder ist in der Lösung, vorzugsweise der kolloidalen Lösung, in einer Menge im Bereich von 10 bis 25 Gew.-%, vorzugsweise 12 bis 20 Gew.-%, besonders bevorzugt 14 bis 18 Gew.-%, vorhanden.

Der in Schritt a) ebenfalls eingesetzte Binder kann in einer weiteren Ausführungsform ein Al-freier und Si-haltiger Binder (Al-frei bedeutet: <0,1 Gew.-% Al in der Gesamtzusammensetzung des Binders) sein. Der Al-freie und Si-haltige Binder ist vorzugsweise Siliciumdioxid. Der Al-freie und Si-haltige Binder liegt weiterhin bevorzugt nicht in fester Form, sondern in Form einer kolloidalen Dispersion, besonders bevorzugt einem Kieselsol, vor. Das Lösemittel, indem der Al-freie und Si-haltige Binder, vorzugsweise das Siliciumdioxid, dispergiert vorliegt, ist in einer bevorzugten Ausführungsform Wasser. Der Al-freie und Si-haltige Binder, vorzugsweise das Siliciumdioxid, ist in der Dispersion in einer Menge im Bereich von 7 bis 50 Gew.-%, vorzugsweise 12 bis 42 Gew.-%, besonders bevorzugt 20 bis 35 Gew.-% vorhanden. Die mittlere Partikelgröße des Al-freien und Si-haltigen Binders, vorzugsweise des Siliciumdioxids kann, insbesondere in der Dispersion, 5 bis 20, vorzugsweise 6 bis 10 nm betragen (kann mittels Lichtstreumethoden ermittelt werden). Die Viskosität der Dispersion mit dem Al-freien und Si-haltigen Binder, vorzugsweise dem Siliciumdioxid, kann im Bereich von 1 bis 50 mPas, vorzugsweise 5 bis 25 mPas liegen. Weiterhin bevorzugt kann die Dispersion mit dem Al-freien und Si-haltigen Binder, vorzugsweise dem Siliciumdioxid, einen pH-Wert im Bereich von 7 bis 12, vorzugsweise 8 bis 10 aufweisen. Die Dichte der Dispersion mit dem Al-freien und Si-haltigen Binder, vorzugsweise dem Siliciumdioxid, beträgt vorzugsweise 1 bis 1,3 g/cm$^3$, besonders bevorzugt1,1 bis 1,25 g/cm$^3$.

Als Nickelquelle kann in Schritt a) eine Lösung einer Nickelverbindung aus einer Nickelverbindung, wobei prinzipiell jede lösliche Nickelverbindung eingesetzt werden kann. Darunter fallen Nickelnitrat ($Ni(NO_3)_2$), Nickelacetat ($Ni(ac)_2$), Nickelacetylacetonat ($Ni(acac)_2$), Nickelsulfat ($NiSO_4$), Nickelcitrat oder Nickelcarbonat ($NiCO_3$). Bevorzugt sind Nickelnitrat ($Ni(NO_3)_2$), Nickelsulfat ($NiSO_4$) oder Nickelcarbonat ($NiCO_3$). Die Nickel-Lösung ist eine wässrige oder ammoniakalische Lösung, vorzugsweise eine ammoniakalische Lösung. Eine ammoniakalische Lösung ist in diesem Zusammenhang als eine wässrige Lösung, welche mit Ammoniak versetzt wurde, zu verstehen. Die Lösung einer Nickelverbindung kann Nickel in einer Menge im Bereich von 1 bis 20 Gew.-%, vorzugsweise 5 bis 15 Gew.-%, enthalten, jeweils bezogen auf das Gesamtgewicht der Lösung. In einer bevorzugten Ausführungsform wird als Nickel-Lösung eine ammoniakalische $Ni(CO_3)$-Lösung, genannt NiHAC-Lösung (es bildet sich in der Lösung ein Nickelhexamincarbonat-Komplex ($[Ni(NH_3)_6]CO_3$)), verwendet, die einen Nickelgehalt im Bereich von 1 bis 20 Gew.-%, vorzugsweise 5 bis 15 Gew.-% aufweist.

Bei der Herstellung der Mischung in Schritt a) werden auch Wasser und optional Ammoniak eingesetzt. Wasser und das optionale Ammoniak können jeweils einzeln oder als Mischung bei der Herstellung der Mischung hinzugegeben werden. Es ist aber auch möglich, dass Binder und/oder Nickelquelle als wässrige bzw. ammoniakalische Lösung bei der Herstellung der Mischung hinzugegeben werden und optional weiteres Wasser und Ammoniak einzeln oder als Mischung hinzugegeben werden. Beim Verfahren gemäß der vorliegenden Erfindung muss zu keinem Zeitpunkt mehr Flüssigkeit hinzugefügt werden, als notwendig ist, um die feuchte Mischung zu bilden.

In einer besonders bevorzugten Ausführungsform der vorliegenden Erfindung wird in Schritt a) eine Mischung umfassend amorphes Aluminiumsilikat, welches 10 bis 65 Gew.-% Aluminiumoxid und 35 bis 90 Gew.-% Siliciumdioxid umfasst, optional einen Al-haltigen und Si-freien Binder oder einen Al-freien und Si-haltigen Binder, eine ammoniakalische $Ni(CO_3)$-Lösung als Nickelquelle, wobei der Flüssiganteil in der Mischung das 1,5-fache des TPV (total pore volume) des Aluminiumsilikats und des Binders (sofern vorhanden), vorzugsweise das 1,3-fache des TPV, besonders bevorzugt das 1,25-fache des TPV nicht überschreitet hergestellt und granuliert. Der für die Granulation notwendige Flüssiganteil kann zum Teil während der Granulation durch Zugabe von z. B. Nickellösung, Wasser oder wässrige Ammoniaklösung angepasst werden. Soll eine höhere Nickel-Konzentration erreicht werden, kann eine zusätzliche Nickelquelle als Feststoff hinzugefügt werden. Bei der Granulation entstehen Partikel unterschiedlicher Größe, wobei die granulierten Partikel erfindungsgemäße vorzugsweise im Bereich von 0,5 bis 5 mm. Es entstehen jedoch auch Partikel, die außerhalb dieses Bereichs sind. Diese können als Recyclematerial zurückgeführt werden, wobei Partikel, die nach Granulation zu klein sind, direkt in die Granulation zurückgeführt werden und Partikel, die zu groß sind, abgetrennt, gemahlen und dann erst in die Granulation zurückgeführt werden.

Nach der Herstellung der Mischung in Schritt a), wonach die Mischung aufgrund des Flüssiganteils als feuchte Mischung vorliegt, erfolgt in Schritt b) eine hydrothermale Behandlung. Die erfindungsgemäß hergestellte Mischung, die weiter als feuchte Mischung vorliegt, kann vorher jedoch auch einer Formgebung unterworfen worden. In diesem Fall findet die hydrothermale Behandlung an der aus der Formgebung erhaltenen Mischung statt. Die hydrothermale Behandlung wird erfindungsgemäß bei einer an den Behälter angelegten Temperatur im Bereich von 75 °C bis 180 °C, vorzugsweise 80 °C bis 150 °C durchgeführt und kann in einem offenen oder geschlossenen Behälter durchgeführt werden. Die Temperatur in der feuchten Mischung steigt dabei vorzugsweise auf 70 bis 110 °C, vorzugsweise 75 bis 95 °C an. Die Temperaturangabe bezieht sich dabei auf die feuchte Mischung oder die Temperatur, die in dem Behälter vorliegt. Unter einem offenen Behälter in Zusammenhang mit der hydrothermalen Behandlung wird verstanden, dass der Behälter nicht fest verschlossen ist, sondern eine Öffnung aufweist. Beispiele dafür wären Ventile oder Überdruckventile, die ein Bersten des Behälters verhindern sollen, oder die zumindest teilweise Abdeckung der Öffnung mit einer teildurchlässigen Membran. Dadurch kann die Trocknungsrate klein gehalten, aber auch sich möglicherweise bildende Überdrücke, die z. B. für den Behälter kritisch sein können, verhindert werden.

Die hydrothermale Behandlung ist der Kern des vorliegenden Verfahrens und klar von der nachfolgenden Trocknung in Schritt c) abzugrenzen. Bei der hydrothermalen Behandlung ist die Trocknungsrate (Masseverlust an Wasser und optional Ammoniak pro Zeiteinheit (z. B. min)) signifikant geringer als bei der Trocknung in Schritt c). Dies wird durch geeignete Bedingungen und/oder Geräte erreicht, beispielsweise Art und Bauweise des Behälters. Die hydrothermale Behandlung in Schritt b) kann auch in einem geschlossenen Behälter erfolgen, wodurch die Trocknungsrate bezogen auf das Volumen des Behälters gleich oder Nahe 0 ist. Wird die hydrothermale Behandlung in einem geschlossenen Behälter durchgeführt, steigt der Druck durch das verdampfende Wasser und optional den verdampfenden Ammoniak. Aus Sicherheitsgründen kann es in diesem Fall erforderlich sein, dass der geschlossene Behälter ein Überdruckventil aufweist, um zu verhindern, dass der Druck zu hoch wird.

Die Dauer der hydrothermalen Behandlung muss nicht zwangsläufig auf eine bestimmte Zeit eingeschränkt werden. Es sollte sich durch die hydrothermale Behandlung zumindest der erfindungsgemäß gewünschte Anstieg der Festigkeit und ein Anstieg der Katalysatoraktivität einstellen, der durch entsprechende Messung (s. Beispiele) überprüft werden kann. Auf der anderen Seite sollte die hydrothermale Behandlung schon aus wirtschaftlichen Gründen nicht zu lange durchgeführt werden, um Energie und damit Kosten zu sparen. In einer bevorzugten Ausführungsform hat es sich als vorteilhaft erwiesen, wenn die Dauer der hydrothermalen Behandlung im Bereich von einer Stunde bis 24 Stunden liegt.

Zwar kann die hydrothermale Behandlung in Schritt b) in einem geschlossenen Behälter erfolgen. Es ist aber auch möglich, dass die Mischung aus Schritt a) nach optionaler Formgebung während der hydrothermalen Behandlung mit einem Gas um- oder durchströmt wird. Es ist vorteilhaft, wenn man die Menge, des Gases, das die Mischung aus Schritt a) nach optionaler Formgebung während der thermischen Behandlung um- oder durchströmt, begrenzt. Die Raumgeschwindigkeit, des Gases, das die Mischung während der thermischen Behandlung pro Stunde um- oder durchströmt, sollte vorzugsweise unterhalb des 50-fachen Volumens der Mischung liegen.

Die erfindungsgemäß hergestellte Mischung, die weiter als feuchte Mischung vorliegt, kann vor der Trocknung einer Formgebung unterworfen worden. Dabei kann aus der Mischung eine für den herzustellenden Oligomerisierungskatalysator geeignete Form gebracht werden, beispielsweise in Form eines Granulats oder in Form von Tabletten. Die Formgebung ist aber nur optional, weil es grundsätzlich möglich ist den Katalysator auch in Form eines Pulvers einzusetzen. Somit kann die in Schritt a) hergestellte Mischung entweder vor der hydrothermalen Behandlung in Schritt b) einer Formgebung unterworfen wird, vorzugsweise einer Granulation, einer Tablettierung, einer Extrusion oder einer Matrizenpressung, besonders bevorzugt einer Granulation. Es ist aber auch möglich, dass die in Schritt b) einer hydrothermalen Behandlung unterworfene Mischung erst vor der Trocknung in Schritt c) einer Formgebung unterworfen wird, vorzugsweise einer Granulation, einer Tablettierung, einer Extrusion oder einer Matrizenpressung, besonders bevorzugt einer Granulation.

Nach der hydrothermalen Behandlung in Schritt b) oder nach der eine Formgebung, wenn dies nicht bereits vor der hydrothermalen Behandlung ist, wird die jeweils erhaltene Mischung in Schritt c) einer Trocknung unterworfen. Dazu können bekannte Geräte wie beispielsweise geeignete Öfen oder in kontinuierlicher Fahrweise beispielsweise Bandtrockner oder ähnliches verwendet werden. Entsprechende Vorrichtungen sind dem Fachmann bekannt. Die Trocknungstemperatur kann im Bereich von 80 bis 250 °C, vorzugsweise im Bereich von 100 bis 220 °C liegen. Im Gegensatz zur hydrothermalen Behandlung ist bei der Trocknung in Schritt c) ein Verdampfen der Flüssigkeit (Wasser und optional Ammoniak) notwendig. Die Trocknungsrate ist daher höher als bei der hydrothermalen Behandlung in Schritt b), vorzugsweise mindestens doppelt so hoch wie bei der hydrothermalen Behandlung in Schritt b).

In einer bevorzugten Ausführungsform wird die Trocknung so durchgeführt, dass das erhaltene getrocknete Material einen Trockenverlust bei 110 °C (LOD) von maximal 10 Gew.-%, vorzugsweise maximal 5 Gew.-% aufweist. Der Trockenverlust bei 110 °C wird dabei wie folgt bestimmt: Zu Beginn wird die untersuchte Materialprobe gewogen (m[Einwaage]); anschließend wird die Materialprobe für eine Dauer von 6 h in einem Trockenschrank bei 110 °C getrocknet; danach lässt man die Materialprobe im Exsikkator abkühlen und wiegt sie abschließend erneut (m[Auswaage]); der Trockenverlust bei 110 °C (LOD) ergibt sich dann wie folgt:

$$LOD = 100\,\% * (m[\text{Einwaage}] - m[\text{Auswaage}]) / m[\text{Einwaage}].$$

Nach der Trocknung in Schritt c) erfolgt die Kalzination der getrockneten Mischung zu Herstellung des fertigen Oligomerisierungskatalysators. Dabei kann die Mischung in einem geeigneten Ofen, vorzugsweise in einem Luftstrom, in einem Stickstoffstrom oder einer Kombination daraus erhitzt werden. Der Luft- oder Stickstoffstrom kann im Gleichstrom oder im Gegenstrom durchgeleitet werden. Dem Stickstoffstrom kann während der Kalzination Luft hinzugefügt werden. Die Kalzinationstemperatur kann 400 bis 800 °C, vorzugsweise 450 bis 750 °C, besonders bevorzugt 550 bis 650 °C betragen. Diese Temperatur kann über mehrere Stunden, vorzugsweise 0.25 bis 20 Stunde, besonders bevorzugt 0.5 bis 10 Stunden gehalten werden, bevor das Granulat abgekühlt wird.

Beispiele

Herstellung der Oligomerisierungskatalysatoren

1464 g eines Alumosilikats (Davicat® O 701), 293 g einer kolloidalen Dispersion von Siliziumdioxidpartikeln (Köstrosol 0830 AS) und 2240 g einer Nickelquelle (NiHAC-Lösung, Nickelgehalt zwischen ca. 12 Gew.-%) wurden in einem Intensivmischer granuliert. Nach diesem Ansatz wurden zwei identische Granulationsansätze hergestellt. Im ersten Ansatz wurden insgesamt 3765 g feuchte Granulate erhalten. Die Ausbeute an feuchten Granulaten mit einer Größe von 1,00 bis 3,15 mm lag bei 77 %. Im zweiten Ansatz wurden 3782 g feuchte Granulate erhalten, wobei die Ausbeute an feuchten Granulaten mit einer Größe von 1,0 bis 3,15 mm bei 73 % lag. Für die Präparation der beiden Katalysatormuster wurde die Fraktion 1,0 bis 3,15 mm der beiden Granulationsansätze miteinander vermischt. Das TPV von Aluminiumsilikats und Binder zusammen beträgt gemäß DIN66134 1,1 ml/g. Der Flüssiganteil in der Mischung beträgt daher das 1,26-fache des TPV.

Herstellung des Katalysators 1 mit hydrothermaler Behandlung (erfindungsgemäß)

Hydrothermale Behandlung

In neun 500 ml Glasflaschen wurden jeweils 200 g der wie vorgenannt hergestellten feuchten Granulate gegeben und die Flaschen mit einem Membrandeckel verschlossen. Die Flaschen wurden in einen auf 115 °C aufgeheizten Umlufttrockenschrank (Firma Vötsch VTU 60/60) mit Frischluftzufuhr gestellt und dort für 6 h belassen. Der Massenverlust betrug in dieser Zeit 25 %. Nach der Behandlung wurden 1300 g Granulate erhalten.

Trocknung

Die Granulate wurde nach der hydrothermalen Behandlung auf einem Siebboden in einen Umlufttrockenschrank der (Firma Vötsch VTU 60/60) mit Frischluftzufuhr bei 115 °C für innerhalb von 1 bis 2 Stunden getrocknet und verblieb über Nacht im Ofen. Das Material wurde nach insgesamt 16 Stunden aus dem Ofen genommen. Der Masseverlust betrug insgesamt ca. 50%, wobei die Trocknungsrate bei der Trocknung höher war als bei der hydrothermalen Behandlung.

Kalzination

Die Kalzination der Granulate erfolgte in einem mit Stickstoff (GHSV = 1000 $h^{-1}$) durchströmten Reaktor. Der Reaktor wurde von Raumtemperatur mit einer Aufheizrate von 1 K/min auf 550 °C aufgeheizt. Die Temperatur von 550 °C wurde für 10 h gehalten. Danach wurde die Heizung ausgestellt und der Reaktor abgekühlt. Die Abkühlung des Reaktors erfolgte weiterhin bei einer GHSV von 1000 $h^{-1}$, aber in einem Gasstrom aus $N_2$ und ca. 5000 ppm Luft.
Herstellung des Katalysators 2 ohne hydrothermale Behandlung (nicht erfindungsgemäß)

Trocknung

1800 g der wie vorgenannt hergestellten feuchten Granulate wurden auf einem Siebboden in einem Umlufttrockenschrank der Firma (Vötsch VTU 60/60) in einen Umlufttrockenschrank mit Frischluftzufuhr bei 115 °C innerhalb von 1 bis 2 Stunden getrocknet und über Nacht im Ofen stehen gelassen. Das Material wurde nach insgesamt 16 Stunden aus dem Ofen genommen. Der Masseverlust betrug insgesamt ca. 49 %.

Kalzination

Die Kalzination der Granulate erfolgte in einem mit Stickstoff (GHSV = 1000 $h^{-1}$) durchströmten Reaktor. Der Reaktor wurde von Raumtemperatur mit einer Aufheizrate von 1 K/min auf 550 °C aufgeheizt. Die Temperatur von 550 °C wurde für 10 h gehalten. Danach wurde die Heizung ausgestellt und der Reaktor abgekühlt. Die Abkühlung des Reaktors erfolgte weiterhin bei einer GHSV von 1000 $h^{-1}$, aber in einem Gasstrom aus $N_2$ und ca. 5000 ppm Luft.

Messung bestimmter Eigenschaften

An den so erhaltenen Oligomerisierungsktatalysatoren wurden sowohl Untersuchungen zu Festigkeit als auch zu deren Performance in der Oligomerisierung durchgeführt.

Beispiel 1 - Bestimmung der BCS (bulk crush strength)

Eine abgewogene Portion des zu untersuchenden Katalysators wird in einen Metallzylinder festgelegter Abmessung gegeben. Ein mit variabler Kraft beaufschlagbarer Stempel presst die Schüttung zusammen. Der bei verschiedenen Drücken entstehende Feinanteil wird abgesiebt und jeweils in Summe gewogen. Die Bruchfestigkeit ist definiert als derjenige Druck in MPa, bei dem ein Feinanteil von 0,5 Gew.-% auftritt. Für die eigentliche Messung wurden 20 ml der gesiebten Probe (> 0,425 - < 6 mm) auf 0,1 g genau gewogen und in den Zylinder gefüllt. Durch wiederholtes stoßartiges Aufsetzen des Zylinders auf eine feste Unterlage wird eine gleichmäßig dichte Packung der Probe erreicht. Die Probe wurde nun mit 5 ml Stahlkugeln bedeckt und der Stempel oben draufgestellt, um die Messung durchzuführen. Die Ergebnisse der Messung sind in Tabelle 1 dargestellt.

Tabelle 1: Ergebnisse der BCS-Messung

| Katalysator | BCS |
| --- | --- |
| 1 (erfindungsgemäß) | 1,2 MPa |

(fortgesetzt)

| Katalysator | BCS |
|---|---|
| 2 (nicht erfindungsgemäß) | 0,3 MPa |

Es ist ersichtlich, dass aufgrund der hydrothermalen Behandlung ein deutlicher festerer Katalysator mit höher BCS hergestellt werden konnte.

Beispiel 2 - Abrasion

Eine Teil des zu untersuchenden Katalysators (ca. 100 g) wird einer speziellen Drehtrommel (Umwälzapparat nach ASTM D 4058-96) werden unter definierten Bedingungen umgewälzt (Umdrehungsgeschwindigkeit 60 min$^{-1}$; Umwälzzeit 30 min) und der entstandene Feinanteil über ein Prüfsieb (Maschenweite 0,850 mm, nach DIN 4188 1977-10) abgetrennt (Siebdauer 5 min, Amplitude 1,2 mm/g). Durch Differenzwägung der Formkörper vor und nach der Umwälzung wird der Abrieb bestimmt (in Anlehnung am ASTM D 4058-96, wobei das Material nicht wie in der Vorschrift zusätzlich kalziniert wurde, um eine schnellere Messung mit ausreichender Genauigkeit durchführen zu können). Die Ergebnisse sind in Tabelle 2 gezeigt: Der Abrieb ist hier also der prozentuale Massenverlust.

Tabelle 2: Ergebnisse der Abriebsmessung

| Katalysator | Abrieb (Gew.-%) |
|---|---|
| 1 (erfindungsgemäß) | 1,1 |
| 2 (nicht erfindungsgemäß) | 15,2 |

Es ist deutlich zu erkennen, dass aufgrund der hydrothermalen Behandlung ein deutlicher festerer Katalysator hergestellt werden konnte.

Beispiel 3 - Einsatz in der Oligomerisierung

Es wurden jeweils etwa 350 g des jeweiligen Katalysators in ein Metallrohr mit einem Innendurchmesser vom 23 mm eingefüllt. Vor und hinter dem Katalysator wurden Glasperlen mit einem Durchmesser von 2 mm gegeben, die als Vorheiz- bzw. Abkühlphase dienen. Die Oligomerisierung wurde unter Verwendung von einem Buten/Butan-Gemisch mit 60 % n-Butenen bei 30 bar und einer Belastung von 4,5 g/h Buten pro Gram Katalysator durchgeführt, wobei die Reaktionstemperatur zwischen 90 °C und 110 °C variiert wurde. Die Produkte wurden gaschromatographisch auf den Umsatz an Butenen und die Linearität der Octene analysiert.

Die in Abhängigkeit von der Temperatur erzielten Umsätze und Selektivitäten für Katalysator 1 (erfindungsgemäß) und den nicht erfindungsgemäßen Katalysator 2 sind in den Tabellen 3 und 4 angegeben. Im Hinblick auf die Selektivität wird nachfolgend auf den Iso-Index abgestellt. Die Linearität eines Oligomerisierungsprodukts bzw. der entstandenen Dimere wird durch den Iso-Index beschrieben und stellt einen Wert für die mittlere Anzahl an Methylverzweigungen im Dimer dar. So tragen (für wie im vorliegenden Fall Buten als Edukt) z.B. n-Oktene mit 0, Methylheptene mit 1 und Dimethylhexene mit 2 zum Iso-Index einer C8-Fraktion bei. Je niedriger der Iso-Index ist, umso linearer sind die Moleküle in der jeweiligen Fraktion aufgebaut. Der ISO-Index errechnet sich nach der folgenden allgemeinen Formel:

$$\frac{\text{(einfach verzweigte Dimere (Gew.-\%)} + 2 \times \text{zweifach verzweigte Dimere (Gew.-\%))}}{100}$$

Tabelle 3: Umsätze und Selektivitäten bei der Oligomerisierung von Butenen unter Verwendung von Katalysator 1

| Belastung (Zulauf C4-Olefine in g/h pro Masse Katalysator in g) als WSHV: 4,5 h$^{-1}$ | | | |
|---|---|---|---|
| | Temperatur | Umsatz | Selektivität |
| | | bezogen auf C4-Olefine | Iso-Index |
| Katalysator 1 (erfindungsgemäß) | 90 °C | 48,7 | 0,96 |
| | 100 °C | 46,8 | 0,94 |
| | 110 °C | 45,6 | 0,93 |

Tabelle 4: Umsätze und Selektivitäten bei der Oligomerisierung von Butenenunter Verwendung von Katalysator 2

| Belastung (Zulauf C4-Olefine in g/h pro Masse Katalysator in g) als WSHV: 4,5 h$^{-1}$ | | | |
|---|---|---|---|
| | Temperatur | Umsatz | Selektivität |
| | | bezogen auf C4-Olefine | Iso-Index |
| | 90 °C | 42,1 | 1,00 |
| Katalysator 2 (nicht erfindungsgemäß) | 100 °C | 40,0 | 0,99 |
| | 110 °C | 38,6 | 0,97 |

Es ist ersichtlich, dass aufgrund der hydrothermalen Behandlung ein Katalysator hergestellt werden konnte, der eine bessere Katalysatorperformance, d. h. einen höheren Umsatz und eine geringeren Iso-Index, zeigt.

**Patentansprüche**

1. Verfahren zur Herstellung eines Nickel-enthaltenden Oligomerisierungskatalysators, wobei das Verfahren die folgenden Schritte umfasst:

   a) Herstellung einer Mischung umfassend amorphes Aluminiumsilikat welches 1 bis 45 Gew.-% Aluminiumoxid und 55 bis 99 Gew.-% Siliciumdioxid umfasst, optional einen Al-haltigen und Si-freien oder Al-freien und Si-haltigen Binders, eine Nickelquelle, die eine Nickelverbindung umfasst, und Wasser, wobei der Flüssiganteil in der Mischung das 1,5-fache des TPV (total pore volume) des Aluminiumsilikats und des optionalen Binders, vorzugsweise das 1,3-fache des TPV, besonders bevorzugt das 1,25-fache des TPV nicht überschreitet, wobei das TPV mittels Stickstoffadsorption gemäß DIN 66134 bestimmt wird;
   b) hydrothermale Behandlung der so hergestellten Mischung, wobei die thermische Behandlung bei einer Temperatur im Bereich von 75 °C bis 180 °C, vorzugsweise 80 °C bis 150 °C erfolgt;
   c) Trocknen des aus Schritt b) erhaltenen Mischung, wobei die Trocknungsrate in Schritt c) höher ist als in Schritt b);
   d) Kalzinieren der in Schritt c) getrockneten Mischung zur Herstellung des Oligomerisierungskatalysators.

2. Verfahren nach Anspruch 1, wobei die Nickelverbindung aus der Gruppe, bestehend aus Nickelnitrat (Ni(NO$_3$)$_2$), Nickelacetat (Ni(ac)$_2$), Nickelacetylacetonat (Ni(acac)$_2$), Nickelsulfat (NiSO$_4$), Nickelcitrat oder Nickelcarbonat (NiCO$_3$), ausgewählt wird.

3. Verfahren nach Anspruch 1 oder 2, wobei die in Schritt a) hergestellte Mischung Ammoniak umfasst und das Ammoniak in Schritt a) als Teil der Nickelquelle oder als AmmoniakWasser-Mischung zugegeben wird.

4. Verfahren nach Anspruch 3, wobei als Nickelquelle eine ammoniakalische Ni(CO$_3$)-Lösung eingesetzt wird.

5. Verfahren nach einem der vorhergehenden Ansprüche, wobei in Schritt a) als Al-haltiger und Si-freier Binder ein oxidisches Aluminiummaterial oder als Al-freier und Si-haltiger Binder Siliciumdioxid eingesetzt wird.

6. Verfahren nach einem der vorhergehenden Ansprüche, wobei in Schritt a) als Al-haltiger und Si-freier Binder Aluminiumoxid, Aluminiumhydroxid oder Aluminiumoxid-hydroxid eingesetzt wird.

7. Verfahren nach einem der vorhergehenden Ansprüche, wobei die in Schritt a) hergestellte Mischung vor der hydrothermalen Behandlung in Schritt b) einer Formgebung unterworfen wird, vorzugsweise einer Granulation, einer Tablettierung, einer Extrusion oder einer Matrizenpressung.

8. Verfahren nach einem der vorhergehenden Ansprüche, wobei die in Schritt b) einer hydrothermalen Behandlung unterworfenen Mischung vor der Trocknung in Schritt c) einer Formgebung unterworfen wird, vorzugsweise einer Granulation, einer Tablettierung, einer Extrusion oder einer Matrizenpressung.

9. Verfahren nach einem der vorhergehenden Ansprüche, wobei die Dauer der hydrothermalen Behandlung im Bereich von einer Stunde bis 24 Stunden beträgt.

10. Verfahren nach einem der vorhergehenden Ansprüche, wobei die hydrothermale Behandlung in einem offenen oder geschlossenen Behälter durchgeführt wird.

11. Verfahren nach einem der vorhergehenden Ansprüche, wobei die Trocknung in Schritt b) bei einer Temperatur im Bereich von 80 bis 250 °C, vorzugsweise im Bereich von 100 bis 220 °C erfolgt.

12. Verfahren nach einem der vorhergehenden Ansprüche, wobei die Trocknung in Schritt c) so durchgeführt wird, dass das erhaltene getrocknete Material einen Trockenverlust bei 110 °C (LOD) von maximal 10 Gew.-%, vorzugsweise maximal 5 Gew.-% aufweist.

13. Verfahren nach einem der vorhergehenden Ansprüche, wobei die Trocknungsrate in Schritt c) doppelt so hoch wie in Schritt b) ist.

14. Verfahren nach einem der vorhergehenden Ansprüche, wobei die Kalzination in Schritt c) bei einer Temperatur zwischen 400 °C und 800 °C durchgeführt wird.

15. Verfahren nach einem der vorhergehenden Ansprüche, wobei die Kalzination in Schritt c) im Luftstrom, im Stickstoffstrom oder in einer Kombination daraus durchgeführt wird.

Europäisches
Patentamt

European
Patent Office

Office européen
des brevets

# EUROPÄISCHER RECHERCHENBERICHT

**Nummer der Anmeldung**

**EP 22 21 1067**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 10 850 261 B2 (EVONIK OPERATIONS GMBH [DE]) 1. Dezember 2020 (2020-12-01) * Beispiele * ----- | 1-15 | INV. B01J21/12 B01J23/755 B01J35/00 |
| Y | US 10 633 302 B2 (EVONIK DEGUSSA GMBH [DE]; EVONIK OPERATIONS GMBH [DE]) 28. April 2020 (2020-04-28) * Beispiele * ----- | 1-15 | B01J37/02 B01J37/03 B01J37/08 B01J37/10 |

**RECHERCHIERTE SACHGEBIETE (IPC)**

B01J

Der vorliegende Recherchenbericht wurde für alle Patentansprüche erstellt

| Recherchenort | Abschlußdatum der Recherche | Prüfer |
|---|---|---|
| München | 5. April 2023 | de Cauwer, Robby |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...............................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 22 21 1067

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-04-2023

| Im Recherchenbericht angeführtes Patentdokument | Datum der Veröffentlichung | Mitglied(er) der Patentfamilie | Datum der Veröffentlichung |
|---|---|---|---|
| US 10850261 B2 | 01-12-2020 | AR 116142 A1 | 07-04-2021 |
| | | CA 3036488 A1 | 14-09-2019 |
| | | CN 110270359 A | 24-09-2019 |
| | | EP 3549669 A1 | 09-10-2019 |
| | | JP 2019171369 A | 10-10-2019 |
| | | KR 20190108508 A | 24-09-2019 |
| | | MY 189106 A | 25-01-2022 |
| | | SG 10201902132T A | 30-10-2019 |
| | | TW 201938266 A | 01-10-2019 |
| | | US 2019283003 A1 | 19-09-2019 |
| | | ZA 201901487 B | 23-12-2020 |
| US 10633302 B2 | 28-04-2020 | CA 3049521 A1 | 25-01-2020 |
| | | CN 110776390 A | 11-02-2020 |
| | | EP 3599230 A1 | 29-01-2020 |
| | | JP 2020015721 A | 30-01-2020 |
| | | KR 20200011887 A | 04-02-2020 |
| | | MY 191614 A | 03-07-2022 |
| | | SG 10201906736P A | 27-02-2020 |
| | | TW 202007678 A | 16-02-2020 |
| | | US 2020031732 A1 | 30-01-2020 |
| | | ZA 201904795 B | 31-08-2022 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3542898 A1